(19) Europäisches Patentamt
European Patent Office
Office européen des brevets

(11) Numéro de publication : **0 129 448**
**B1**

(12) **FASCICULE DE BREVET EUROPÉEN**

(45) Date de publication du fascicule du brevet : **10.02.88**

(51) Int. Cl.[4] : **C 07 D231/22**, C 07 D231/26, C 22 B 3/00, C 01 G 3/00

(21) Numéro de dépôt : **84400700.5**

(22) Date de dépôt : **10.04.84**

(54) **Procédé d'extraction sélective du cuivre utilisant les 4-acyl(3H)-pyrazol-3-ones.**

(30) Priorité : **19.04.83 FR 8306393**

(43) Date de publication de la demande :
**27.12.84 Bulletin 84/52**

(45) Mention de la délivrance du brevet :
**10.02.88 Bulletin 88/06**

(84) Etats contractants désignés :
**BE CH DE FR GB LI**

(56) Documents cités :
**CH-A- 605 796**
**FR-A- 2 277 826**
**GB-A- 1 474 282**
**CHEMICAL ABSTRACTS, vol. 89, no. 8, 21 août 1978, réf. no. 63102t, page 209, Columbus Ohio (US);**
**CHEMICAL ABSTRACTS, vol. 92, no. 16, avril 1980, réf. no. 136129f, page 477, Columbus Ohio (US);**
**Chemical Abstracts 94 128219 (1980)**
**Chemical Abstracts 93 51311 (1979)**
**Chemical Abstracts 92885p (1969)**
**Le dossier contient des informations techniques présentées postérieurement au dépôt de la demande et ne figurant pas dans le présent fascicule.**

(73) Titulaire : **SOCIETE NATIONALE DES POUDRES ET EXPLOSIFS**
**12, quai Henri IV**
**F-75181 Paris Cedex 04 (FR)**

(72) Inventeur : **Gautier, Jean-Claude**
**1 rue Brossolette**
**F-94480 Ablon (FR)**
Inventeur : **Lecolier, Serge**
**3 allée des Cartelines**
**F-91510 Janville-sur-Juine (FR)**
Inventeur : **Soriaux, Claude**
**24 rue des Epis**
**F-91540 Mennecy (FR)**
Inventeur : **Chevalier, Sammy**
**143 boulevard Lefebvre**
**F-75015 Paris (FR)**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

EP 0 129 448 B1

Jouve, 18, rue St-Denis, 75001 Paris, France

**Description**

La présente invention concerne l'extraction sélective du cuivre.

L'invention concerne plus particulièrement l'extraction sélective du cuivre de minerais ou de semi-produits concentrés en contenant, par une technique d'extraction liquide/liquide.

Durant les vingt dernières années, des recherches importantes ont été faites pour la valorisation de minerais de plus en plus pauvres (à environ 1 % de cuivre), difficiles à concentrer ou trop complexes pour être traités par les procédés classiques de la pyrométallurgie.

La mise au point d'agents d'extraction (ci-après dénommés extractants) du cuivre et à haute capacité de charge a permis de développer des procédés d'extraction par solvant.

Plusieurs familles d'extractants organiques ont été proposées pour réaliser une concentration des solutions de lixiviation par extraction liquide/liquide. Il s'agit, pour les plus connus, d'agents chélatants de type oximes hydroxylées, parmi lesquels sont tout particulièrement préconisés ceux mis sur le marché par General Mills sous la dénomination générale de série LIX, et ceux commercialisés respectivement par Shell sous la dénomination SME 529 et par ICI sous la dénomination ACORGA P 5000, ou d'agents chélatants de type hydroxyquinoléine, tels que par exemple ceux commercialisés par Ashland Chemical sous les dénominations KELEX 100 et KELEX 120.

Actuellement, 15 % de la production mondiale de cuivre, soit 1,2 MT, sont obtenus par hydrométallurgie avec l'aide de ces agents d'extraction. L'extraction à l'échelle industrielle se fait alors principalement à partir des milieux sulfuriques de dissolution du minerai au moyen d'extractants mis en solution dans des diluants organiques tels que des hydrocarbures aliphatiques ou aromatiques à haut point d'inflammabilité tels que les kérosènes, les naphtas et analogues. Parmi les produits commerciaux les plus utilisés, on peut citer le SOLVESSO 150 et l'ESCAID 100 de la Société ESSO.

Les inconvénients des extractants utilisés de nos jours sont essentiellement :

1) pour des extractants de la série des LIX, notamment le LIX 64 N qui est le plus développé des produits de cette série :

— une faible capacité de charge en cuivre, en dépit d'une très bonne sélectivité vis-à-vis de Ni et Co, ce qui oblige à opérer avec des volumes de phase organique et aqueuse dans un rapport élevé ou avec une concentration exagérément élevée en extractant ; dans un cas comme dans l'autre, il en découle qu'il faut investir plus qu'il n'est normal soit pour l'installation d'extraction, soit pour l'extractant à mettre en œuvre, avec pourtant, au bout du compte, un résultat encore insuffisant,

— une légère extraction concomitante du fer qui nécessite de retraiter la solution aqueuse de réextraction avant la phase d'électrolyse qui est la phase classique de purification du métal extrait,

— la nécessité d'opérer à une température inférieure à 40 °C, le LIX 63 présent à raison de 1 % dans le LIX 64 N étant instable au-delà de cette température,

2) pour les autres extractants de structure voisine que sont le produit commercialement dénommé SME 529 et ceux de la série des ACORGA (voir plus haut),

— bien qu'ils donnent des coefficients d'extraction plus élevés que les produits de la série des LIX et qu'ils présentent des cinétiques d'extraction et de réextraction plus intéressantes que ceux-ci, on a toutefois à déplorer une réextraction très difficile, d'où il s'ensuit un taux résiduel de cuivre en phase aqueuse trop élevée (voir J. A. Tumilty, Advance in extractive metallurgy, 1977, p. 123),

— on a ainsi été conduit à ajouter des proportions assez importantes d'un agent modifiant (en pratique du 4-nonylphénol), mais l'amélioration de la réextraction qui est susceptible d'en résulter a pour contrepartie une baisse du coefficient d'extraction,

3) pour les extractants du type KELEX, et notamment le KELEX 100, utilisés pour les minerais sulfurés qui donnent des solutions plus concentrées en cuivre (de l'ordre de 30 à 50 g/l), on rajoute du 4-nonylphénol pour éviter la formation d'une troisième phase.

Les travaux menés au cours de ces denières années pour l'extraction en milieu sulfurique des métaux non ferreux, et essentiellement du cuivre, ont ainsi porté, pour la plupart, sur des essais d'amélioration des systèmes existants et visaient à remédier tant bien que mal aux défauts connus de ces systèmes, et cela par des essais d'additifs à de tels systèmes visant

— soit à améliorer les performances de l'extractant principal du point de vue de la capacité de charge, de la sélectivité et de la cinétique d'extraction,

— soit à empêcher la formation d'un précipité à l'interface ou d'une troisième phase.

On a d'autre part décrit dans le brevet DD-A-142 059 un procédé de récupération des métaux, et plus particulièrement du plomb, du zinc et du cuivre sans sélectivité, par lessivage de minerais complexes au moyen d'une solution d'agents d'extraction choisis parmi des agents complexants de type polydentates dissous dans des solvants organiques, tels que des acides dialkyldithiophosphoriques, des 4-acyl pyrazolones ou des dithiocarbamates, en solution dans des hydrocarbures chlorés, des hydrocarbures aliphatiques ou aromatiques, des alcools ou des cétones.

Dans ce procédé, les minerais ne sont pas traités au préalable par des solutions alcalines ou acides. Il ne s'agit pas d'une extraction liquide/liquide, mais d'une attaque du minerai concassé par une solution organique d'un agent complexant et ensuite, après séparation du résidu solide, de l'extraction des métaux à partir de ladite solution organique au moyen d'une solution d'un acide minéral dilué (2N).

Ce procédé conduit à une extraction non sélective et oblige à recourir à une étape additionnelle de retraitement pour que puisse être obtenu pour chacun des métaux extraits ensemble.

Le pouvoir chélatant des acylpyrazolones vis-à-vis des métaux est connu :

— MATSUI, dans Bull. Inst. Chem. Res., Kyoto Univ., Vol. 57, N° 5-6, 1979, décrit l'extraction de l'europium de ses solutions aqueuses à l'aide de solutions dans le chloroforme ou le benzène de 4-halogénoacyl 5-pyrazolones dans un but analytique, et indique notamment que la 1-phényl 3-méthyl 4-benzoyl 5-pyrazolone se complexe avec de nombreux métaux.

— PATEL, dans Int. Solvent Extr. Conf., 1980, 3, Paper 80-190, 6 pp, Assoc. Ing. Univ. Liège (CA 94 : 128219 m), enseigne que l'extraction du nickel, avec la 1-phényl 3-méthyl 4-benzoylpyrazolone comme agent d'extraction, est supérieure à celle du cobalt et du cuivre.

— ARORA, dans Symp. Solvent Extr. Met., 1979, III A6/1-III A6/9 (CA 93 : 51311 e), enseigne que le fer (III) est quantitativement extrait, à pH = 2, par les acylpyrazolones.

— ZOLOTOV, dans Zh. Anal. Khim., 1969, 24(1), 20-5 (CA 92885 p), enseigne notamment que le cuivre, nickel, cobalt et fer ainsi que d'autres métaux sont quantitativement extraits par la 1-phényl 3-méthyl 4-benzoyl 5-pyrazolone.

GB 1 474 282 et CH 605796 décrivent des acylpyrazolones et les chélates métalliques correspondants, ainsi que l'application de ces composés à la stabilisation, vis-à-vis notamment des rayonnements, de matières organiques polymérisables.

Le certificat d'auteur russe n° 452178 indique que la présence de groupe —NH—N= améliore la sélectivité de l'extraction du cuivre par des composés hétérocycliques, mais seuls les pyrazoles et les triazoles 1,2,4 sont mentionnés et seul le 3,5 di-n-propyl-4-éthylpyrazole est exemplifié. Dans les complexes correspondants, le cuivre n'est pas lié à deux atomes d'oxygène, comme c'est le cas pour les complexes métalliques des bêta dicétones, en particulier pour les acylpyrazolones précitées.

Le but de la présente invention est l'extraction sélective, et avec un haut rendement, du cuivre à partir de minerais ou de semi-produits concentrés en contenant, par une technique d'extraction liquide/liquide.

Le problème ainsi posé est résolu par un procédé d'extraction liquide/liquide comprenant une attaque préalable du minerai ou du semi-produit concentré par un agent de lixiviation acide en milieu aqueux, l'acide sulfurique de préférence, et selon lequel on extrait le cuivre de la solution d'attaque par une extraction liquide/liquide utilisant au moins une 2-phényl 4-acyl 5-alkyl ou phényl(3H)-pyrazol-3-one appropriée, en solution organique, les groupes phényles pouvant être substitués ou non par des groupes alkyles.

Le procédé conforme à l'invention s'est avéré procurer de façon inattendue une sélectivité d'extraction par rapport au fer et un rendement d'extraction du cuivre tout à fait exceptionnels, une capacité d'extraction meilleure que celles des techniques classiques, une plus grande facilité et un moindre coût de mise en œuvre eu égard à la possibilité d'éviter ou au moins de limiter en deçà de ce qui était indispensable jusqu'ici l'adjonction de composés à action modificatrice tels que le nonylphénol.

On a trouvé selon l'invention que ces résultats, ainsi que d'autres que la suite du présent texte pourra révéler, sont atteints grâce à un procédé qui constitue le premier objet de la présente invention et selon lequel on réalise une extraction liquide/liquide du cuivre à partir d'une solution aqueuse sulfurique d'attaque du minerai ou du semi-produit concentré à traiter, en utilisant au moins un agent d'extraction de type 2-phényl 4-acyl 5-alkyl ou phényl(3H)-pyrazol-3-one dans un solvant organique apte à diluer et dissoudre cet agent d'extraction et, autant que possible, le complexe que celui-ci forme avec le cuivre à extraire.

Plus précisément, l'agent d'extraction utile conformément à l'invention répond à la formule :

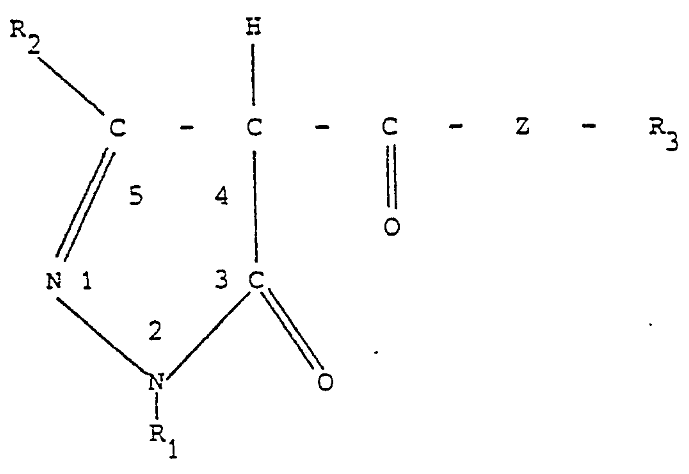

ce composé β-dicarbonylé pouvant tout aussi bien exister sous l'une des deux formes céto-énolyques :

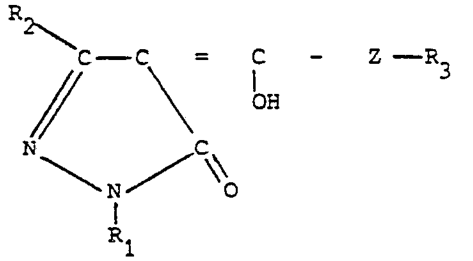
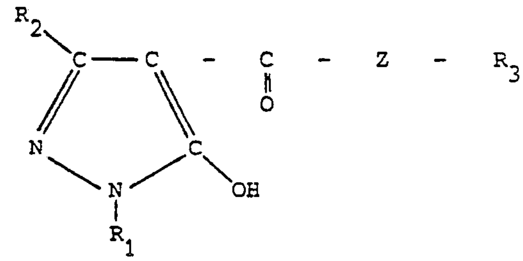

— où $R_1$ représente un groupe phényle, éventuellement substitué par un ou plusieurs groupes alkyles contenant chacun 1 à 12 atomes de carbone

— où $R_2$ représente un groupe alkyle ramifié ou non contenant 1 à 12 atomes de carbone, un groupe phényle éventuellement substitué par un ou plusieurs groupes alkyles contenant chacun 1 à 12 atomes de carbone

— où Z représente un groupe alkylène linéaire ou ramifié, saturé ou insaturé contenant 1 à 21 atomes de carbone

— et où $R_3$ représente H, un (2-phényl 5-alkyl(3H)-pyrazolyl-3-one) carbonyl-4, ou un (2-phényl 5-phényl(3H)-pyrazolyl-3-one) carbonyl-4 formant ainsi un bis acyl pyrazolone de formule :

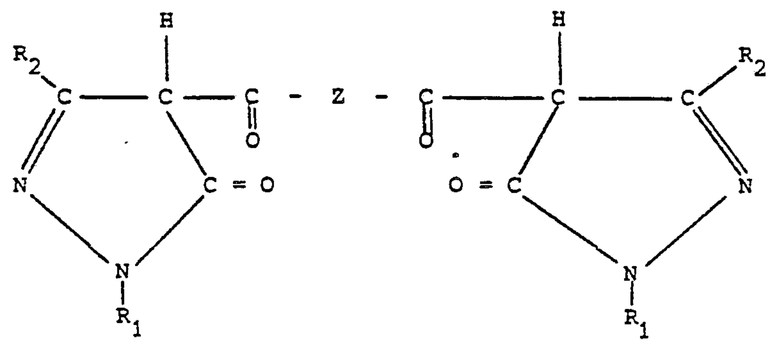

les phényles pouvant être substitués ou non par des groupes alkyles.

Selon une forme de mise en œuvre avantageuse de l'invention, $R_1$ représente le groupe phényle et $R_2$ un groupe alkyle de 1 à 5 atomes de carbone, de préférence le groupe méthyle ; selon une autre forme avantageuse de l'invention, $R_1$ représente un groupe phényle substitué par un groupe alkyle de 8 à 12 atomes de carbone.

Enfin, Z représente de préférence un groupe alkylène de 6 à 17 atomes de carbone et plus particulièrement l'éthyl-1-pentylène ; le undécylène ou l'heptadécylène, avec $R_3 = H$, et un groupe $-(CH_2)_4-$ lorsque $R_3$ représente un groupe pyrazolyl carbonyl tel que défini plus haut.

Pour plus de clarté, il convient de préciser que l'on a choisi de désigner présentement ces produits comme étant des dérivés de 4-acyl(3H)-pyrazol-3-ones, et cela en quasi-conformité avec la nomenclature officielle en vigueur depuis 1972, alors que la nomenclature utilisée avant cette date affectait aux produits de ce type la dénomination de 4-acyl-2-pyrazolin-5-ones. Il s'agit évidemment des mêmes produits.

Certains de ces composés sont nouveaux.

L'invention a donc également pour objet les produits nouveaux rentrant dans le cadre de la formule susdite, et plus particulièrement les suivants :

— 2-phényl 4-(2-éthylhexanoyl) 5-méthyl(3H)-pyrazol-3-one
— 2-phényl 4-(isononanoyl) 5-méthyl(3H)-pyrazol-3-one
— di 1,6-(2-phényl 5-méthyl 4-pyrazolyl-3-one) hexane 1,6-dione
— 2-phényl 4(undécénoyl) 5-méthyl(3H)-pyrazole-3-one
— 2-phényl 4-(oléoyl) 5-phényl(3H)-pyrazol-3-one
— di-1,10-(2-phényl 5-phényl 4-pyrazolyl-3-one) décane 1,10 dione.

Ces composés peuvent être préparés à partir de produits connus par des techniques connues de l'homme de métier comme, par exemple, par le procédé décrit par Jensen dans Acta Chemica Scandinavica, 13, 1668 (1959) selon lequel on fait réagir le chlorure d'acide de formule H—Z—COCl, dans le cas des monopyrazolones, ou ClOCZCOCl, dans le cas des dipyrazolones, sur l'hétérocycle non substitué en position 4 et en présence d'hydroxyde de calcium.

Conformément à l'invention, un solvant organique est utilisé pour diluer et dissoudre l'agent d'extraction et le complexe que ce dernier forme avec le cuivre ; il doit être pratiquement insoluble dans l'eau et ne pas perturber l'action de l'agent d'extraction. Ce solvant organique doit, de préférence, avoir un point d'inflammabilité élevé.

De manière générale, il convient que le solvant organique puisse dissoudre d'environ 20 g/l à environ 750 g/l et de préférence d'environ 50 g/l à environ 200 g/l de l'agent d'extraction.

Des solvants organiques efficaces sont les hydrocarbures aliphatiques ou aromatiques à haut point

d'inflammabilité, tels que les kérosènes, les naphtas et analogues.

Parmi ces solvants, on recommande tout particulièrement les produits disponibles dans le commerce sous la dénomination SOLVESSO et diffusés par la Société ESSO, et plus spécialement celui qui correspond à la dénomination commerciale SOLVESSO 150 et qui est un solvant pétrolier aromatique.

Le processus d'extraction selon l'invention a lieu par mise en contact du solvant organique contenant l'agent d'extraction avec la solution aqueuse d'agent de lixiviation contenant le cuivre, de préférence une solution sulfurique et les autres espèces métalliques et résultant de l'attaque du minerai. La mise en contact des deux phases que sont la phase aqueuse et la phase organique peut se faire par les techniques d'extraction par solvant bien connues de l'homme du métier (extraction continue ou discontinue, emploi de mélangeurs-décanteurs ou de colonnes d'extraction, etc... ; la circulation des fluides se fait de préférence à contre-courant).

Le rapport des volumes des phases aqueuse et organique dépend de l'appareillage utilisé, de la concentration en agent d'extraction dans la phase organique et de la composition de la phase aqueuse à traiter. Un rapport Vaq/Vorg. compris entre 3/1 et 1/3 environ est souhaitable.

La température à laquelle on effectue la mise en contact des phases et la séparation de celles-ci n'est pas un facteur primordial ; elle est simplement liée au point d'inflammabilité du solvant utilisé ; une température comprise entre 20 et 60 °C convient en pratique.

Il est apparu que, pour certaines des acylpyrazolones préconisées selon l'invention, il peut se produire une précipitation du complexe formé entre le cuivre et l'agent d'extraction, au bout d'un certain laps de temps. On a trouvé, et cela constitue une variante du procédé selon l'invention, qu'on peut alors retarder cette précipitation sans pour autant inhiber l'action de l'agent d'extraction en ajoutant à la solution de cet agent d'extraction dans le solvant organique une proportion de 2 à 20 % environ en volume et de préférence 10 % en volume, de 4-nonylphénol ou d'un équivalent.

La phase aqueuse soumise au procédé conforme à l'invention peut être de deux types ; en effet, suivant la nature des minerais ou concentrés de minerais à traiter, et notamment des minerais cuprifères à traiter, on distingue deux types de solutions sulfuriques pour l'extraction du cuivre par voie hydrométallurgique :

— les solutions d'acide sulfurique issues de minerais oxydés, à faible concentration en cuivre (1 à 10 g/l) contenant du fer, du cobalt et du nickel à des teneurs variables et dont le pH est compris entre 1 et 3 ;

— les solutions d'acide sulfurique issues de minerais sulfurés, à forte concentration en cuivre (15 à 40 g/l) contenant aussi de fortes proportions de fer, nickel et cobalt et dont le pH est compris entre 1 et 3.

A des pH plus élevés, les solutions seraient moins stables ; à des pH moins élevés, le coefficient d'extraction serait moins élevé.

L'invention est illustrée plus concrètement ci-après, en référence à des formes préférées de réalisation et à des exemples non limitatifs de mise en œuvre.

Les propriétés d'extraction des moyens préconisés selon l'invention ont été comparées à celles de produits actuellement utilisés industriellement aux mêmes fins, à savoir : le LIX 64 N (qui comprend 1 % de LIX 63)

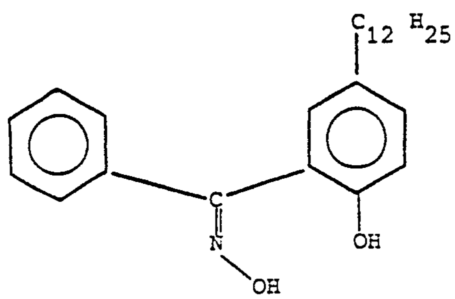

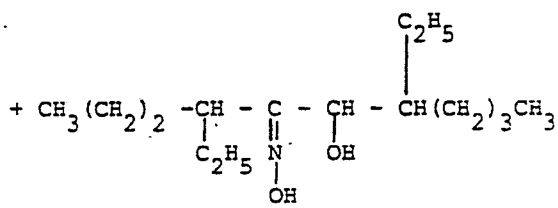

(1 %)

pour des solutions aqueuses sulfuriques à faible teneur en cuivre (environ 3 g/l) ;

— le KELEX 100

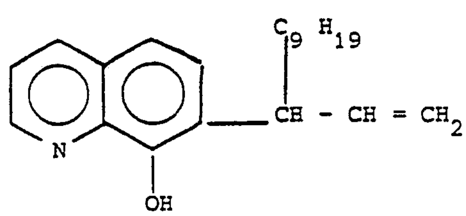

pour des solutions aqueuses sulfuriques à forte teneur en cuivre, provenant de minerais cuprifères sulfurés.

Les tests comparatifs d'extraction correspondants ont été effectués sur des solutions synthétiques

représentatives des compositions industrielles à traiter habituellement et formulées comme suit :

| Composition des solutions | |
|---|---|
| représentatives de minerais à faible teneur en Cu = phase aqueuse de type A | représentatives de minerais sulfurés = phase aqueuse de type B |
| $H_2SO_4$ diluée | $H_2SO_4$ diluée |
| Cu 3 g/l | Cu 23 g/l |
| Fe 3 g/l | Fe 6 g/l |
| | Ni 18 g/l |
| | Co 1 g/l |
| (quantité $H_2SO_4$ suffisante pour pH = 1 et 2,5) | (quantité $H_2SO_4$ suffisante pour pH = 1 et 2) |

D'après une étude préliminaire portant sur les diluants possibles répondant à la définition qui en a été donnée plus haut, on a été conduit à préférer le solvant pétrolier aromatique commercialisé sous la dénomination SOLVESSO 150.

A titre de variante, trouvant son utilité essentiellement dans les cas où il convient de combattre la formation (intervenant en général après plusieurs heures, par exemple après 12 à 24 heures) d'un précipité plus ou moins important dans la phase organique, on a trouvé avantageux d'ajouter également au milieu organique d'extraction d'environ 2 à environ 20 %, et de préférence environ 10 %, en volume de nonylphénol.

Les autres tests effectués sont relatés ci-après en référence aux figures des planches de dessins annexées, dans lesquelles :

— Figure 1 représente l'influence de la concentration en agent d'extraction 2-phényl 4-lauroyl 5-méthyl(3H) pyrazol-3-one dans du SOLVESSO 150 portée en abscisse, ainsi que celle du pH de la phase aqueuse à traiter (phase aqueuse de type A) sur l'extraction (% du Cu extrait porté en ordonnée) ;

— Figure 2 représente les isothermes d'extraction obtenues par traitement d'une solution aqueuse sulfurique de type A à pH 2,5, respectivement :

1) Pour la courbe en trait pointillé, avec du produit commercial LIX 64 N à 15 % dans du diluant commercialisé sous la dénomination ESCAID 100 ;

2) Pour la courbe en trait continu, avec de la 2-phényl 4-lauroyl 5-méthyl(3H) pyrazol-3-one à 50 g/l dans du SOLVESSO 150 + 10 ml/100 ml volume de nonylphénol ;

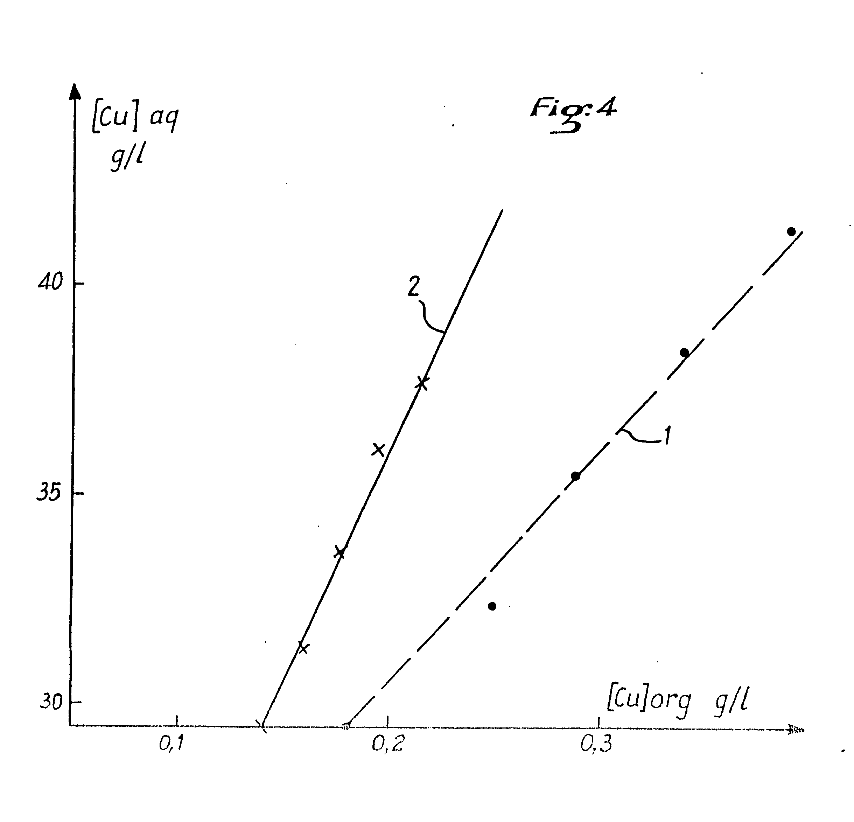

— Figures 3 et 4 représentent les isothermes de réextraction obtenus lors de mise en contact successives d'une même phase aqueuse sulfurique avec une nouvelle phase organique chargée en cuivre.

Dans le cas de la figure 3, la phase aqueuse n'est pas initialement chargée en cuivre.

Dans le cas de la figure 4, la charge en cuivre de la phase aqueuse est initialement de 30 g/l, ce qui simule une phase aqueuse à la sortie du circuit d'électrolyse du cuivre, recyclée comme solution aqueuse de strippage, c'est-à-dire utilisée pour réextraire le cuivre contenu dans la phase organique. La phase organique chargée en cuivre contient comme agent d'extraction dans les troix exemples de la figure 3, respectivement la 2-phényl 4-(2-éthylhexanoyl 5-méthyl(3H)-pyrazol-3-one (courbe 3), du LIX 64 N (courbe 1) et de la 2-phényl 4-lauroyl 5-méthyl(3H) pyrazol-3-one (courbe 2), et dans les exemples de la figure 4, respectivement la 2-phényl 4-(2-éthylhexanoyl) 5-méthyl(3H) pyrazol-3-one courbe 2) et le LIX 64 N (courbe 1).

Ces tests dont les résultats sont rapportés sur les figures susdites, auxquelles il est utile de se reporter, montrent que :

1. En ce qui concerne la concentration optimale en agent d'extraction ainsi que le pH de la phase aqueuse le mieux adapté pour qu'on atteigne le maximum d'extraction :

— dans le cas d'une solution à faible teneur en cuivre :

— à pH 2,5 il n'est pas nécessaire d'utiliser une concentration supérieure à 50 g/l pour atteindre l'épuisement des phases aqueuses avec un nombre limité d'étages,

— par comparaison avec l'agent d'extraction commercial LIX 64 N, les performances de la 2-phényl 4-lauroyl 5-méthyl(3H)-pyrazol-3-one en solution dans le SOLVESSO (concentration 50 g/l) sont équivalentes à celles du LIX 64 N à la concentration de 136 g/l dans du diluant ESCAID 100 (se reporter au tableau I et aux courbes correspondantes de la figure 2).

— dans le cas d'une solution aqueuse sulfurique à forte teneur en cuivre, par comparaison avec le KELEX 100 à la concentration de 15 % en volume (soit 143 g/l) dans le SOLVESSO 150 additionné de 20 %

de nonylphénol, les performances des acylpyrazolones (2-phényl 4-lauroyl [et 4-(2-éthyl hexanoyl)] 5-méthyl(3H) pyrazol-3-one) sont du même ordre de grandeur à concentration équivalente et même encore à une concentration légèrement inférieure (soit 120 à 130 g/l).

(Voir tableau II et figure 3 courbe 1 pour le LIX, 2 pour la lauroylpyrazolone et 3 pour l'hexanoyl.)

2. Les isothermes d'extraction tracées de façon à permettre de connaître la capacité de l'agent d'extraction à saturation (voir figure 2) sont pratiquement confondues pour la 2-phényl 4-lauroyl 5-méthyl (3H) pyrazol-3-one et le LIX 64 N, ce qui confirme l'équivalence des performances entre la « lauroylpyrazolone » à seulement 50 g/l et le LIX 64 N à la concentration de 15 % en volume, soit 136 g/l.

Les tests effectués ont permis de calculer les capacités d'extraction suivantes :

| extractant | mg de cuivre par g d'agent d'extr. | efficacité relative des pyrazolones et du LIX 64 N |
|---|---|---|
| 4-lauroyl-P | 80 | 2,66 |
| 4-stéaroyl-P | 70 | 2,33 |
| 4-(2-éthyl)hexanoyl-P | 90 | 2,83 |
| 4-nonanoyl-P | 104 | 3,46 |
| LIX 64 N | 30 | 1 |

P = 2-phényl 5-méthyl(3H) pyrazol-3-one

On peut en conclure que, pour une quantité équivalente, les 2-phényl 4-acyl 5-méthyl(3H) pyrazol-3-ones ont une capacité d'extraction environ trois fois supérieure à celle du LIX 64 N.

3. Les courbes isothermes de réextraction obtenues pour des tests comparatifs de réextraction par contacts successifs d'une même phase aqueuse sulfurique avec une nouvelle phase organique chargée en espèces métalliques extractibles, sont linéaires, ce qui prouve que la saturation de la phase aqueuse n'est pas atteinte (figures 3 et 4).

Les isothermes sont proches pour la 2-phényl 4-lauroyl 5-méthyl pyrazol-3-one et le produit de type LIX.

La réextraction est légèrement plus facile dans le cas de la 2-phényl 4-(2-éthyl hexanoyl) 5-méthyl(3H) pyrazol-3-one. La figure 4 met notamment bien en évidence, dans un cas représentatif des conditions industrielles d'utilisation (phase aqueuse chargée initialement en cuivre) que la réextraction du cuivre est plus efficace dans le cas de la 2-phényl 4-(2-éthylhexanoyl) 5-méthyl(3H) pyrazol-3-one que dans le cas du LIX 64 N.

En ce qui concerne d'autre part la sélectivité de l'extraction, c'est-à-dire la séparation du Cu et des autres métaux Fe, Ni, Co, des dosages effectués dans le raffinat (phase aqueuse épuisée) et dans la phase organique (et dont les résultats ressortent des tableaux I, II et IV ci-dessous), ont montré que la sélectivité est meilleure pour la grande majorité des produits selon l'invention qu'en utilisant comme extractants les produits du commerce, soit le LIX 64 N ou celui dénommé KELEX 200.

En particulier, on a calculé, dans le tableau IV, la sélectivité Cu/Fe après 10 minutes d'extraction. Cette sélectivité est égale aux rapports des constantes d'extraction

$$K_{Cu}/K_{Fe} = [Cu]\ org/[Cu]\ aq : [Fe]\ org/[Fe]\ aq$$

où [M] org représente la concentration du métal M dans la phase organique et [M] aq la concentration du métal M dans la phase aqueuse.

Puisque les concentrations en fer et en cuivre sont initialement les mêmes, une autre manière d'exprimer la sélectivité est d'effectuer le rapport [Cu] org : [Fe] org.

Cette sélectivité est confirmée par les résultats du dosage du fer et du cuivre dans les solutions aqueuses résultant de la réextraction sulfurique ($H_2SO_4$ 1N) (tableau III ci-dessous) et du dosage de ces mêmes éléments dans la phase organique correspondante.

En effet, en présence de l'acylpyrazolone, on extrait pratiquement 10 fois moins de fer qu'en présence de LIX 64 N, alors que les quantités de cuivre extraites sont pratiquement les mêmes.

La sélectivité vis-à-vis du nickel et du cobalt est encore meilleure. En effet, ces métaux ne sont pas extraits par la 2-phényl 4-lauroyl 5-méthyl(3H) pyrazol-3-one en solution dans le SOLVESSO 150

(concentration 40 g/l), à partir d'une solution aqueuse sulfurique où ils sont présents à une concentration de 3 g/l environ.

Cette sélectivité est encore confirmée par les résultats du traitement d'une solution sulfurique à forte teneur en cuivre (23 g/l), contenant en outre 6 g/l de Fe, 18 g/l de Ni et 1 g/l de Co, par la 2-phényl 4-(2-éthyl hexanoyl) 5-méthyl(3H) pyrazol-3-one dans du diluant SOLVESSO 150 et à la concentration de 0,42 mole par litre.

La phase organique chargée contenait (voir exemple 10 ci-dessous) :

| | | | | |
|---|---|---|---|---|
| Cu | 8 | g/l | soit | 34,8 % d'extraction |
| Ni | 0,15 | g/l | soit | 0,8 % d'extraction |
| Co | < 0,001 | g/l | soit | < 0,1 % d'extraction |
| Fe | 0,06 | g/l | soit | < 1 % d'extraction |

L'exemple 11 ci-dessous concerne quant à lui une comparaison entre la 2-phényl 4-lauroyl 5-méthyl(3H) pyrazol-3-one et le produit du commerce dénommé KELEX 100, et démontre encore la meilleure sélectivité obtenue dans l'extraction du Cu en présence de fer, de nickel et de cobalt avec la pyrazolone selon l'invention.

L'invention est illustrée plus en détail dans les exemples ci-après, qui ne la limitent aucunement.

### Exemple 1

Extraction du cuivre de solutions à basse teneur, de pH 2,5, par la 2-phényl 4-lauroyl 5-méthyl(3H) pyrazol-3-one.

Une solution aqueuse sulfurique, à pH 2,5, contenant 2,9 g/l de cuivre ($Cu^{2+}$) et 3,2 g/l de fer est mise en contact avec une solution de 2-phényl 4-lauroyl 5-méthyl(3H) pyrazol-3-one à 100 g/) dans le SOLVESSO 150, sans additif de type nonyphénol.

L'extraction est effectuée, volume à volume, en ampoule à décanter, à 20 °C, avec agitation mécanique pendant une demi-heure. Le pH de la solution aqueuse à l'équilibre est alors mesuré.

Après décantation les teneurs en cuivre et en fer de la phase aqueuse résiduelle (dite raffinat) sont mesurées par absorption atomique. Les teneurs en métaux de la phase organique sont calculées par différence.

Les résultats obtenus sont les suivants :

| | |
|---|---|
| — pH à l'équilibre | 1,4 |
| — Cu extrait | 99,7 % du Cu initial |
| — Fe extrait | 3 % environ du Fe initial. |

### Exemple 2

Extraction du cuivre de solutions à basse teneur, de pH = 1, par la 2-phényl 4-lauroyl 5-méthyl(3H) pyrazol-3-one.

Une solution aqueuse sulfurique, à pH 1, contenant 2,9 g/l de cuivre $Cu^{2+}$ et 3,2 g/l de fer est mise en contact, volume à volume, avec une solution de 2-phényl 4-lauroyl 5-méthyl(3H) pyrazol-3-one à 100 g/l dans le SOLVESSO 150, sans addition de nonylphénol.

L'extraction et les dosages sont effectués comme dans l'exemple n° 1.

Les résultats sont les suivants :

| | |
|---|---|
| — pH à l'équilibre | 0,8 |
| — Cu extrait | 85,3 % du Cu initial |
| — Fe extrait | 0 % environ. |

### Exemple 3

Extraction du cuivre d'une solution à basse teneur, de pH 2,5, par la 2-phényl 4-(2-éthyl hexanoyl) 5-méthyl(3H) pyrazol-3-one.

Une solution aqueuse sulfurique, à pH 2,5, contenant 3 g/l de cuivre $Cu^{2+}$ et 3,2 g/l de fer est mise en contact, volume à volume, avec une solution de 2-phényl 4-(2-éthyl hexanoyl) 5-méthyl(3H) pyrazol-3-one à 42 g/l dans le SOLVESSO 150.

L'extraction et les dosages sont effectués comme dans l'exemple n° 1.

Les résultats sont les suivants :

| | |
|---|---|
| — pH à l'équilibre | 1,5 |
| — Cu extrait | 82 % du Cu initial |
| — Fe extrait | 0 % environ |

## Exemple 4

Extraction du cuivre d'une solution à basse teneur, de pH = 2,5, par la 2-phényl 4-stéaroyl 5-méthyl(3H)-pyrazol-3-one.

Une solution aqueuse sulfurique à pH 2,5, contenant 2,9 g/l de cuivre $Cu^{2+}$ et 3,2 g/l de fer est mise en contact, volume à volume, avec une solution de 2-phényl 4-stéaroyl 5-méthyl(3H) pyrazol-3-one à 62 g/l dans le SOLVESSO 150 avec 10 ml/100 ml de nonylphénol.

L'extraction et les dosages sont effectués comme dans l'exemple n° 1.

Les résultats sont les suivants :

| | |
|---|---|
| — pH à l'équilibre | 1,7 |
| — Cu extrait | 89,6 % du Cu initial |
| — Fe extrait | 0 % environ |

## Exemple 5

Extraction du cuivre d'une solution à basse teneur, de pH 2,3, par la 2,5-diphényl 4-(oléoyl) (3H) pyrazol-3-one.

Une solution aqueuse sulfurique, à pH 2,3, contenant 3,2 g/l de cuivre $Cu^{2+}$ et 3,35 g/l de fer est mise en contact, volume à volume, avec une solution de 2,5 diphényl 4-(oléoyl) (3H) pyrazol-3-one à 50 g/l dans le SOLVESSO 150.

L'extraction est effectuée comme dans l'exemple n° 1. Les dosages sont effectués par mesure des teneurs en cuivre et fer, par absorption atomique, dans les phases aqueuse et organique.

| | |
|---|---|
| — pH à l'équilibre | 1,4 |
| — Cu extrait | 68 % du Cu initial |
| — Fe extrait | 0,3 % du Fe initial |

## Exemple 6

Extraction du cuivre d'une solution à basse teneur de pH 2,3 par la 2-phényl 4-(undécénoyl) 5-méthyl(3H) pyrazol-3-one.

Une solution aqueuse sulfurique, à pH 2,3, contenant 3,13 g/l de cuivre $Cu^{2+}$ et 3,21 g/l de fer est mise en contact, volume à volume, avec une solution de 2-phényl 4-(undécenoyl) 5-méthyl(3H) pyrazol-3-one à 50 g/l dans le SOLVESSO 150, avec 10 % volume à volume de nonylphénol.

L'extraction et les dosages sont effectués comme dans l'exemple n° 5.

| | |
|---|---|
| — pH à l'équilibre | 1,4 |
| — Cu extrait | 68 % du Cu initial |
| — Fe extrait | 1,4 % du Fe initial |

## Exemple 7

Extraction du cuivre d'une solution à basse teneur, de pH 2,3, par la 2-phényl 4-lauroyl 5-n propyl(3H) pyrazol-3-one

Une solution aqueuse sulfurique, à pH 2,3, contenant 3,13 g/l de cuivre $Cu^{2+}$ et 3,21 g/l de fer est mise en contact, volume à volume, avec une solution de 2-phényl 4-lauroyl 5-n propyl(3H) pyrazol-3-one à 50 g/l dans le SOLVESSO 150, avec 10 % volume à volume de nonylphénol.

L'extraction et les dosages sont effectués comme dans l'exemple n° 5.

| | |
|---|---|
| — pH à l'équilibre | 1,45 |
| — Cu extrait | 66 % du Cu initial |
| — Fe extrait | 0,6 % du Fe initial |

## Exemple 8

Extraction du cuivre d'une solution à basse teneur, de pH 2,5, par la 2,5 diphényl 4-(lauroyl) (3H) pyrazol-3-one.

Une solution aqueuse sulfurique, à pH 2,5, contenant 3 g/l de cuivre $Cu^{2+}$ et 3 g/l de fer est mise en contact, pendant 1/2 heure, volume à volume, avec une solution 0,14 M (50,6 g/l) de 2,5 diphényl 4-(lauroyl) (3H) pyrazol-3-one dans le SOLVESSO 150, avec 10 % de nonylphénol volume à volume.

L'extraction et les dosages sont effectués comme dans l'exemple n° 5.

| | |
|---|---|
| — pH à l'équilibre | 1,4 |
| — Cu extrait | 76 % du Cu initial |
| — Fer extrait | 0,7 % du Fe initial |

Exemple 9

Extraction du cuivre d'une solution à moyenne teneur, de pH 2,3, par la 2-phényl 4(éthyl-2 hexanoyl) 5-méthyl(3H) pyrazol-3-one.

Une solution aqueuse sulfurique, à pH 2,3, contenant 6,16 g/l de cuivre $Cu^{2+}$ et 1,24 g/l de fer est mise en contact, pendant 1/2 heure, volume à volume, avec une solution à 100 g/l de 2-phényl 4-(éthyl-2 hexanoyl) 5-méthyl(3H) pyrazol-3-one dans le SOLVESSO 150 avec 2 % de nonylphénol (volume à volume).

L'extraction et les dosages sont effectués comme dans l'exemple n° 5.

| | |
|---|---|
| — pH à l'équilibre | 1,4 |
| — Cu extrait | 76 % du Cu initial |
| — Fer extrait | 1,2 % du Fe initial |

Exemple 10

Extraction du cuivre d'une solution à haute teneur, de pH = 2, par la 2-phényl 4-(2-éthylhexanoyl) 5-méthyl(3H) pyrazol-3-one.

Une solution aqueuse sulfurique, à pH 2, contenant 23 g/l de cuivre, 6 g/l de fer, 18 g/l de nickel et 1 g/l de cobalt est mise en contact avec une solution de 2-phényl 4-(2-éthylhexanoyl) 5-méthyl(3H) pyrazol-3-one à 126 g/l dans le SOLVESSO 150.

L'extraction est effectuée en ampoule à décanter, avec une agitation mécanique pendant une demi-heure. Les éléments sont dosés par absorption atomique soit dans la phase aqueuse résiduelle (raffinat), soit, pour augmenter la sensibilité de la mesure, pour certains dans une phase aqueuse de réextraction ($H_2SO_4 1N$).

Les résultats sont les suivants :

| | |
|---|---|
| — Cu extrait | 34,8 % du Cu initial |
| — Ni extrait | 0,8 % du Ni initial |
| — Fe extrait | 1 % du Fe initial |
| — Co extrait | 0,1 % du Co initial |

Exemple 11

Extraction du cuivre d'une solution à haute teneur, de pH 2, par la 2-phényl 4-lauroyl 5-méthyl(3H) pyrazol-3-one.

Une solution aqueuse sulfurique, à pH 2, contenant 22,6 g/l de cuivre, 6,1 g/l de fer, 19,2 g/l de nickel et 0,8 g/l de cobalt est mise en contact, volume à volume, à 20 °C, avec une solution de 2-phényl 4-lauroyl 5-méthyl(3H) pyrazol-3-one à 150 g/l dans le SOLVESSO 150.

L'extraction est effectuée en ampoule à décanter, avec agitation pendant une demi-heure. Les éléments sont dosés par absorption atomique dans la phase aqueuse résiduelle (raffinat) et les taux d'extraction sont évalués par différence.

Les résultats sont les suivants :

| | |
|---|---|
| — pH final à l'équilibre | 0,9 |
| — Cu extrait | 54,9 % du Cu initial |
| — Fe extrait | 2,1 % du Fe initial |
| — Ni extrait | 1,6 % du Ni initial |
| — Co extrait | 2,5 % du Co initial |

A titre de comparaison, l'extraction de la même solution acide par le KELEX 100 à 143 g/l dans le SOLVESSO 150 (avec 20 ml/100 ml de nonylphénol) donne les résultats suivants :

| | |
|---|---|
| — pH final à l'équilibre | 1,1 |
| — Cu extrait | 45,3 % du Cu initial |
| — Fe extrait | 4,8 % du Fe initial |
| — Ni extrait | 2,7 % du Ni initial |
| — Co extrait | 3,7 % du Co initial |

Exemple 12

Capacité d'extraction de 2-phényl 4-acyl 5-méthyl(3H) pyrazol-3-ones.

Les isothermes d'extraction sont réalisés par contacts successifs de la même phase organique avec une nouvelle phase aqueuse dans les conditions suivantes :

Phase aqueuse : solution sulfurique à pH 2,5 de 3 g/l de cuivre et 3 g/l de fer.

Phase organique initiale solution à 50 g/l de l'acyl pyrazolone dans le SOLVESSO 150 avec 10 ml/100 ml de nonylphénol (sauf pour la 2-phényl 4-(2-éthyl hexanoyl) 5-méthyl(3H) pyrazol-3-one où on n'ajoute pas de nonylphénol).

Les extractions sont effectuées volume à volume, à 20 °C en ampoules à décanter, avec, à chaque fois, un contact d'une demi-heure sous agitation. Le cuivre est dosé par absorption atomique dans les phases aqueuses résiduelles (raffinat) et la concentration en cuivre dans les phases organiques est évaluée par différence.

A partir des isothermes d'extraction, on calcule la capacité de l'extractant à saturation en divisant la charge maximale (en g de métal par litre) appréciée sur l'isotherme par la concentration de l'extractant supposé pur exprimée en g/l.

pour la 2-phényl 4-lauroyl 5-méthyl(3H) pyrazol-3-one : 80 mgCu/g extractant ;
2-phényl 4-stéaroyl 5-méthyl(3H) pyrazol-3-one : 70 mgCu/g
2-phényl 4-(2-éthyl hexanoyl) 5-méthyl(3H)-pyrazol-3-one : 90 mgCu/g
2-phényl 4-isononanoyl 5-méthyl(3H) pyrazol-3-one : 104 mgCu/g

A titre de comparaison, la capacité d'extraction du LIX 64 N, à 136 g/litre dans l'ESCAID 100 est 30 mgCu/g d'extractant.

Exemple 13

Réextraction dans le cas de la 2-phényl 4-lauroyl 5-méthyl(3H) pyrazol-3-one.

La réextraction a été réalisée dans les conditions suivantes : les isothermes de réextraction sont réalisées par contacts successifs d'une même phase aqueuse $H_2SO_4$ (1N) avec une nouvelle phase organique contenant 50 g/l de 2-phényl 4-lauroyl 5-méthyl(3H) pyrazol-3-one dans du SOLVESSO 150 contenant 10 ml/100 ml de nonylphénol et 4 g/l de cuivre ; l'équilibre correspondant aux plus faibles concentrations est obtenu par épuisement de la phase organique résultant du premier contact par une phase aqueuse vierge.

Les contacts ont lieu en ampoules à décanter, à 20 °C pendant une demi-heure. Les concentrations sont déterminées par absorption atomique, à la fois dans les phases aqueuses et dans les phases organiques (après dilution dans l'éthanol pour ces dernières). Les isothermes d'extraction sont rassemblées dans la figure 3, où la courbe 1 concerne le LIX 64 N à 136 g/l dans l'ESCAID 100 et la courbe 2 est relative à la 2-phényl 4-lauroyl 5-méthyl(3H) pyrazol-3-one.

Exemple 14

Réextraction dans le cas de la 2-phényl 4-(2-éthyl hexanoyl) 5-méthyl(3H) pyrazol-3-one.

On a procédé comme indiqué dans l'exemple 13. L'isotherme de réextraction a été établie à partir des résultats obtenus à la suite de contacts successifs (volume à volume) d'une même phase aqueuse $H_2SO_4$(1N) avec une nouvelle phase organique contenant l'extractant à la concentration de 0,14 mole/l, soit 42 g/l, et du cuivre à raison de 3,3 g/l dans du SOLVESSO 150 et sans nonylphénol.

Les contacts avaient lieu en ampoules à décanter, sous agitation, pendant une demi-heure, à température ambiante.

Les dosages du cuivre et du fer ont été effectués par absorption atomique dans les phases aqueuses de réextraction.

L'isotherme tracée est linéaire et donne des valeurs légèrement supérieures à celles avec le LIX 64 N et la 2-phényl 4-lauroyl 5-méthyl(3H) pyrazol-3-one (figure 3, courbe 3).

Exemple 15

Réextraction par une solution aqueuse de strippage à 30 g/l de cuivre. Cas de la 2-phényl 4-(éthyl 2-hexanoyl) 5-méthyl(3H) pyrazol-3-one.

La réextraction a été réalisée dans les conditions suivantes : les isothermes de réextraction sont déterminées par contacts successifs d'une même phase aqueuse ($H_2SO_4$ 3N, Cu 30 g/l) avec une nouvelle phase organique contenant 50 g/l de 2-phényl 4-(éthyl 2-hexanoyl) 5-méthyl(3H) pyrazol-3-one dans du SOLVESSO 150 contenant 10 ml/100 ml de nonylphénol et 3,5 g/l de cuivre.

Les contacts ont lieu en ampoule à décanter, à 20 °C, pendant une demi-heure. Les concentrations sont déterminées par absorption atomique, à la fois dans les phases aqueuses et dans les phases organiques (après dilution dans l'éthanol pour ces dernières). Les isothermes d'extraction sont rassemblées dans la figure n° 4 où la couche 1 concerne le LIX 64 N (à 15 % dans l'ESCAID 100) et la courbe 2 la 2-phényl 4-(éthyl-2 hexanoyl) 5-méthyl(3H) pyrazol-3-one.

Exemple 16

Sélectivité cuivre/fer

La sélectivité cuivre-fer a été étudiée par dosage du fer dans les solutions de réextraction $H_2SO_4$ 1N obtenues comme décrit dans l'exemple 13, la solution aqueuse initiale à basse teneur en cuivre, à pH 2,5, contenant 3 g/l de cuivre et 3 g/l de fer.

Pour les solutions finales les plus chargées en cuivre, on obtient les résultats suivants :

2-phényl 4-lauroyl 5-méthyl(3H) pyrazol-3-one
Cu : 11,9 g/l Fe : 0,032 g/l

2-phényl 4-(2-éthyl hexanoyl) 5-méthyl(3H) pyrazol-3-one
Cu : 11,5 g/l Fe : < 0,01 g/l

LIX 64 N
Cu : 13,8 g/l Fe : 0,310 g/l

Exemple 17

Extraction du cuivre et du fer par les 4-acyl(3H) pyrazol-3-ones. Sélectivité comparative

Toutes les 4-acyl(3H) pyrazol-3-ones sont étudiées suivant le même processus opératoire :

La solution aqueuse de sulfate de cuivre à 3 g/l de cuivre ($Cu^{2+}$) et de sulfate de fer à 3 g/l de fer est ajustée à pH 2,5 par ajout de $H_2SO_4$ sous contrôle potentiométrique.

50 ml de cette solution sont versés dans une cellule thermostatée à 25 °C munie d'une agitation magnétique. Sur cette solution on verse lentement 50 ml de la phase organique d'extraction (0,14 mole de 4-acyl(3H) pyrazol-3-one dans le SOLVESSO 150 avec ou non, suivant les cas, 10 % de nonylphénol (V/V) en évitant toute agitation.

Enfin l'agitation magnétique, préalablement réglée à 750 tpm, est mise en fonction.

Des prélèvements intermédiaires, dans les phases aqueuses et organiques, aux temps 3, 10 et 60 minutes sont effectués pour mesurer, par absorption atomique, les quantités de fer et de cuivre extraites.

Les résultats sont rassemblés dans le tableau IV, exprimés en pourcentage d'extraction par rapport à la quantité initialement présente dans la solution aqueuse.

Exemple 18

Préparation de la 2-phényl 4-(2-éthyl hexanoyl) 5-méthyl(3H) pyrazol-3-one.

On agite un mélange de 1 044 g (6 moles) de 2-phényl 5-méthyl(3H) pyrazol-3-one dans 4 l de dioxanne et on porte la température à 60 °C pour réaliser la dissolution complète de la pyrazolone. On ajoute ensuite 888 g (12 moles) d'hydroxyde de calcium $Ca(OH)_2$ puis en 45 minutes, 876 g (5,4 moles) de chlorure de 2-éthyl hexanoyle. Le milieu s'échauffe jusqu'à 95 °C, on maintient cette température et l'agitation durant 2 heures.

Après refroidissement, le mélange réactionnel est versé dans 8 l d'HCl (2N). Une couche organique se sépare, on ajoute 2 l de toluène pour faciliter la décantation ;

La phase organique est séchée sur sulfate de magnésium et le solvant éliminé sous pression réduite.

Le résidu huileux obtenu avec un rendement de 77 % est rectifié sur un appareil de distillation sous vide et à film mince. Le produit distille à 162 °C sous 0,1 Pa.

— Le rendement en produit purifié est de 61 %.

— L'identification a été réalisée

1 — par RMN(H).

Le spectre de RMN, réalisé en solvant $CDCl_3$, avec comme référence le TMS présente les pics suivants :

— à 2,5 ppm : singulet correspondant au $CH_3$ en position 5 sur le noyau pyrazol,

— entre 0,7 et 3,1 ppm : multiplets attribués au reste alkyl,

— entre 7,2 et 8 ppm : multiplets du noyau aromatique,

— à 14 ppm : singulet correspondant au proton énolique.

2 — par analyse élémentaire

| | | | |
|---|---|---|---|
| — valeurs expérimentales : | C 71,97 % | H 8,39 % | N 9,32 % |
| — valeurs théoriques : | C 71,93 % | H 8,00 % | N 9,46 % |

3 — indice de réfraction $n_d^{20} = 1,5560$

4 — densité à 20 °C : 1,0678

Exemple 19

Préparation de la 2-phényle 4-isononanoyl 5-méthyl(3H) pyrazol-3-one

En suivant le mode opératoire de l'exemple 18, avec les mêmes proportions mais en remplaçant le

chlorure de 2-éthyl hexanoyle par du chlorure d'isononanoyle, on obtient la 2-phényl 4-isononanoyle 5-méthyl(3H) pyrazol-3-one avec un rendement de 75 %. Ce composé distille à 165 °C sous 0,1 Pa. Le spectre RMN tracé dans le $CDCl_3$, avec comme référence TMS, présente les pics :

0,9 à 1,4 ppm(m) ; 2,5 ppm(s) ; 2,6 à 2,8 ppm(m) ;
7,2 à 8,1 ppm(m) ; 13,5 ppm(s).

Exemple 20

Préparation de la di-1,6(2-phényl-5-méthyl 4-pyrazolyl-3-one) hexane-1,6-dione.

0,225 mole (39,15 g) de 2-phényl 5-méthyl pyrazol-3-one est dissoute dans 250 $cm^3$ de dioxane. On ajoute 0,4 mole de $Ca(OH)_2$ (29,6 g) puis rapidement 0,1 mole de chlorure d'adipoyle $ClCO(CH_2)_4COCl$ (18,3 g). On porte au reflux 3/4 d'heure.

Le mélange réactionnel est versé dans 1,2 litre d'acide chlorhydrique 2N. Le produit solide qui précipite est séparé et redissous dans le chlorure de méthylène. La solution organique est séchée sur sulfate de magnésium puis évaporée. On obtient un produit solide de point de fusion 203 °C, et dont le spectre de masse présente un pic molaire (M + 1) à 459.

Exemple 21

Préparation de 2-phényl 4-(oleoyl) 5-phényl(3H) pyrazol-3-one.

0,2 mole, soit 47,2 g, de 2-phényl pyrazol-3-one est dissoute dans 600 $cm^3$ de dioxane à la température de 60 °C. On ajoute 0,4 mole de chaux (29,6 g) puis en 30 minutes 0,2 mole de chlorure d'oleoyle. On élève la température du mélange réactionnel à 95 °C et on laisse sous agitation durant 2 heures.

Le mélange refroidi est versé dans 1 litre d'acide chlorhydrique (2N). Une huile se sépare ; elle est reprise par du chlorure de méthylène. Cette phase organique est séchée, puis le solvant est éliminé sous vide.

On récupère un produit huileux avec un rendement de 84,5 %. La purification a été effectuée en isolant le complexe de cobalt de l'oléoylpyrazolone.

50 g de dérivé brut soit 0,1 mole sont mélangés dans 100 $cm^3$ de méthanol avec 0,055 mole d'acétate de cobalt anhydre soit 9,73 g. Le mélange est porté à reflux durant 30 minutes.

Le sel de cobalt de l'oleoyl pyrazolone précipite dans le méthanol, il est filtré et lavé à l'eau.

Ce sel est placé dans du chlorure de méthylène et sous agitation on introduit de l'acide sulfurique (3N) de façon à détruire le complexe de cobalt et libérer l'oleoyl pyrazolone qui est alors solubilisée dans le chlorure de méthylène.

Cette phase organique est séparée, lavée à l'eau, séchée, et le solvant éliminé sous pression réduite.

L'huile obtenue correspond à l'oleoyl pyrazolone pure dont l'identification a été réalisée par RMN (triplet des protons éthyléniques entre 5,2 et 5,5 ppm).

Exemple 22

Préparation de la 2-phényl 4-(undécénoyl) 5-méthyl(3H) pyrazol-3-one.

En suivant le mode opératoire de l'exemple 18, avec les mêmes proportions, mais en remplaçant le chlorule de 2-éthyl hexanoyl par le chlorure d'undécénoyle, on obtient un dérivé solide qui après recristallisation dans l'hexane a un point de fusion de 40 °C.

Sur le spectre RMN, avec comme référence le TMS et en solvant $CDCl_3$ on identifie les pics suivants :

— 1 singulet à 2,5 ppm correspondant au méthyl en position 5 sur le cycle pyrazol ;
— 1 triplet entre 2,6 et 2,9 ppm correspondant aux protons méthyléniques en $\alpha$ du groupement carbonyle ;
— 1 doublet entre 1,9 et 2,1 ppm attribué aux protons méthyléniques en $\alpha$ de l'insaturation ;
— entre 0,9 et 2,4 multiplets correspondant aux protons restants de la chaîne alkyl ;
— entre 4,8 et 6,1 ppm multiplets attribués aux protons portés par la double liaison ;
— entre 7,1 et 8 ppm multiplets des protons aromatiques ;
— 14,5 ppm singulet provenant du proton énolique.

Exemple 23

Préparation de la di 1,10(2-phényl-5-phényl 4-pyrazolyl-3-one) décane-1,10 dione.

0,2 mole soit 47,2 g de 2-phényl 5-phényl pyrazol-3-one est dissoute dans 600 $cm^3$ de dioxane, à la température de 60 °C. On ajoute 0,4 mole de chaux $Ca(OH)_2$ (29,6 g), puis en 30 minutes 0,09 mole de chlorure de sébacoyle, soit 21,5 g. La température du milieu s'élève à 95 °C ; on la maintient durant 2 heures.

Après refroidissement on verse le mélange réactionnel dans 1 litre d'acide chlorhydrique (2N).

Le solide qui précipite est redissous dans 200 $cm^3$ de chlorure de méthylène.

La solution organique est lavée 2 fois avec 200 $cm^3$ d'$H_2SO_4$(3N) puis à l'eau distillée. Elle est ensuite séchée et le solvant éliminé sous pression réduite.

Le produit solide obtenu, avec un rendement de 49,3 % a un point de fusion de 168 °C et présente en RMN un spectre en accord avec la formule du composé.

Exemple 24

Préparation de la 2-phényl 4-(lauroyl) 5-phényl(3H) pyrazol-3-one.

En suivant le mode opératoire de l'exemple 18, avec les mêmes proportions mais en remplaçant la 2-phényl-5-méthyl(3H) pyrazol-3-one, par la 2-phényl 5-phényl(3H) pyrazol-3-one (Mw = 136,3 g ; PF = 136 °C) et le chlorure de 2-éthyl hexanoyle par le chlorure de lauroyle, on obtient un produit solide qui après recristallisation dans l'hexane a un point de fusion de 69 °C.

— L'identification a été effectuée par RMN.

— La pureté a été contrôlée par analyse élémentaire.

| | Théorie | Mesuré |
|---|---|---|
| C % | 77,51 | 76,4 |
| H % | 8,13 | 8,2 |
| N % | 6,7 | 6,75 |

Exemple 25

Préparation de la 2-phényl 4-(lauroyl) 5-propyl(3H) pyrazol-3-one

Selon le mode opératoire de l'exemple 18, avec les mêmes proportions, mais en remplaçant la 2-phényl 5-méthyl(3H) pyrazol-3-one par la 2-phényl 5-propyl(3H) pyrazol-3-one (Mw = 202 ; PF = 114°) et le chlorure de 2-éthyl hexanoyle par le chlorure de lauroyle on obtient un produit solide qui après recristallisation dans l'hexane a un point de fusion de : 61 °C.

Le spectre de RMN tracé dans le $CDCl_3$ avec comme référence le TMS présente les pics caractéristiques de la 2-phényl 4-(lauroyl) 5-propyl(3H) pyrazol-3-one :

— 0,7 à 2,8 ppm (multiplets des $CH_3$ et $CH_2$)

— 7,2 à 8,1 ppm (multiplets des H aromatiques)

— 14,5 ppm (singulet du proton énolique)

Exemple 26

Affinité des 4-acyl(3H) pyrazol-3-ones pour le cation $Fe^{3+}$

Toutes les 4-acyl(3H) pyrazol-3-ones sont étudiées suivant le même processus opératoire :

Une solution aqueuse de sulfate ferrique $Fe_2(SO_4)_3$ à 7,5 g/l de $Fe^{3+}$ est ajustée à pH2 par ajout éventuel de $H_2SO_4$ sous contrôle potentiométrique.

Cette phase aqueuse est mise en contact, pendant 15 minutes, sous agitation dans une ampoule à décanter, avec une solution de 4-acyl(3H) pyrazol-3-one (0,2 M dans le SOLVESSO 150, sans additif nonylphénol). L'extraction est effectuée, volume à volume, à température ambiante.

Les dosages sont effectués par mesure des teneurs en fer, par absorption atomique, dans les phases aqueuses et organiques.

Les résultats sont rassemblés dans le tableau V. On constate une affinité importante des 4-acyl(3H) pyrazol-3-ones par le fer lorsque $R_1$ est une chaîne aliphatique ou lorsque $ZR_3$ est aromatique. Les 4-acyl pyrazolones correspondantes ne permettent donc pas une extraction sélective du cuivre vis-à-vis du fer.

Exemple 27

Extraction du cuivre et du fer par la 2-phényl 4-thénoyl 5-méthyl(3H) pyrazol-3-one.

L'extraction est conduite comme dans l'exemple n° 17 : une solution 0,14 M de 2-phényl 4-thénoyl 5-méthyl(3H) pyrazol-3-one dans le SOLVESSO 150 sans nonylphénol est mise en contact avec une solution aqueuse sulfurique à 3 g/l de cuivre $Cu^{2+}$ et 3 g/l de fer (pH 2,5).

Après dosage des éléments cuivre et fer dans les 2 phases, les résultats sont les suivants après 3

minutes d'extraction :

— Cu extrait : 71 %

— Fe extrait : 7,9 %

— $[Cu]_{org}$ : $[Fe]_{org}$ ≃ 9, soit une mauvaise sélectivité vis-à-vis du fer.

Exemple 28

Extraction du cuivre et du fer par la 2-n lauryl 4-lauroyl 5-méthyl(3H) pyrazol-3-one

L'extraction est menée comme dans l'exemple n° 17 : une solution 0,14 M de 2-n lauryl 4-lauroyl 5-méthyl(3H) pyrazol-3-one dans le SOLVESSO 150, sans nonylphénol comme additif, est mise en contact avec une phase aqueuse sulfurique à 3 g/l de cuivre $Cu^{2+}$ et 3 g/l de fer (pH 2,5).

Après dosage des éléments cuivre et fer, par absorption atomique, dans les 2 phases, les résultats sont les suivants, après 60 minutes d'extraction :

— Cu extrait : 48,8 %

— Fe extrait : 6,9 %

— $[Cu]_{org}$ : $[Fe]_{org}$ ≃ 7, soit une mauvaise sélectivité vis-à-vis du fer.

Exemple 29

Extraction du cuivre et du fer par la 2-phényl 4-benzoyl 5-méthyl(3H) pyrazol-3-one

Une phase organique contenant la 2-phényl 4-benzoyl 5-méthyl(3H) pyrazol-3-one 0,14 M dans le SOLVESSO 150, avec 10 % de nonylphénol, est mise en agitation, volume à volume, dans une ampoule à décanter, avec une phase aqueuse sulfurique à pH 2,5 contenant 3 g/l de cuivre $Cu^{2+}$ et 3 g/l de fer. L'agitation dure une demi-heure à température ambiante.

Un précipité du complexe avec le cuivre apparaît immédiatement.

Les dosages sont effectués, sur les phases aqueuses, par absorption atomique. Les résultats sont les suivants :

— Cu extrait : 97 %

— Fe extrait : 40 %

— $[Cu]_{org}$ : $[Fe]_{org}$ ≃ 2,5, soit pratiquement aucune sélectivité vis-à-vis du fer.

(Voir Tableaux p. 16 et suivantes)

Tableau I

Influence de la concentration en extractant et du pH de la phase aqueuse sur l'extraction du cuivre et du fer

| Concentration en extractant / phase aqueuse initiale de type A ($H_2SO_4$ dilué) | 2-phényl 4-lauroyl 5-méthyl(3H)pyrazol-3-one | | | | | | | | | LIX 64 N | | |
|---|---|---|---|---|---|---|---|---|---|---|---|---|
| | 50 g/l | | | 100 g/l | | | 150 g/l | | | 15% (V/V).136 g/l | | |
| | pH final | % extrait Cu | % extrait Fe | pH final | % extrait Cu | % extrait Fe | pH final | % extrait Cu | % extrait Fe | pH final | % extrait Cu | % extrait Fe |
| Cu : 2,9 g/l<br>Fe : 3,2 g<br>pH : 1 | 0,8 | 46,5 | ~0 | 0,8 | 85,3 | ~0 | 0,8 | 95,9 | ~6 | 0,8 | 43,5 | ~0 |
| Cu : 2,9 g/l<br>Fe : 3,2 g/l<br>pH ≠ 2,5 | 1,5 | 90,0 | ~0 | 1,4 | 99,7 | ~3 | 1,4 | 99,9 | ~6 | 1,4 | 91,2 | ~6 |

~ mis pour environ

Le diluant utilisé était :

— du SOLVESSO 150 pour la pyrazolone

— le produit dénommé ESCAID 100 pour l'extractant du type LIX.

Tableau II

Influence de la concentration en extractant et du pH de la phase aqueuse sur l'extraction du cuivre et des impuretés : Fe, Co, Ni

| Concentration en extractant / phase aqueuse initiale type B ($H_2SO_4$) diluée | 2-phényl 4-lauroyl 5-méthyl (3H)pyrazol-3-one | | | | | | | | | | | | | | | KELEX 100 | | | | |
|---|---|---|---|---|---|---|---|---|---|---|---|---|---|---|---|---|---|---|---|---|
| | 50 g/l | | | | | 100g/l | | | | | 150g/l | | | | | 15%(V,V). 143 g/l | | | | |
| | pH final | % extrait Cu | Fe | Ni | CO | pH final | % extrait CU | Fe | Ni | Co | pH final | % extrait Cu | Fe | Ni | Co | pH final | % extrait Cu | Fe | Ni | Co |
| Cu : 22,6 g/l<br>Fe : 6,1 g/l<br>Ni : 19,2 g/l<br>Co : 0,8 g/l<br>pH 1 | 0,9 | 13,5 | 0 | 0 | 0 | 0,8 | 34,5 | 0 | 0 | 0 | 0,8 | 56,6 | 17,6 | 16,7 | 11 | 0,7 | 40,7 | 7,9 | 0 | 0 |
| mêmes concentrations<br>pH 2 | 1,5 | 18,2 | 0 | nd | nd | 1,1 | 34,1 | 0 | nd | nd | 0,9 | 54,9 | 2,1 | 1,6 | 2,5 | 1,1 | 45,3 | 4,8 | 2,7 | 3,7 |

Le diluant utilisé était du SOLVESSO 150 pour la pyrazolone et pour le KELEX 100.

Tableau III

| Sélectivité | Concentration dans les solutions aqueuses de ($H_2SO_4$ 1N) réextraction | |
|---|---|---|
| | Cu (g/l) | Fe (g/l) |
| 4-lauroyl-P | 11,9 | 0,032 |
| 4-(2-éthylhexanoyl)P | 11,5 | <0,010 |
| LIX 64 N | 13,8 | 0,310 |

P = 2-phényl 5-méthyl(3H) pyrazol-3-one.
Solution aqueuse initiale, type A : Cu 3 g/l
Fe 3 g/l et pH = 2,5.

Tableau IV — Extraction du cuivre en présence de fer par les 4-acyl(3H) pyrazol-3-ones

| 4-acyl (3H) pyrazol-3-ones | | | Cuivre extrait après | | | Fer extrait après | | | Sélectivité à 10 min $= \frac{KCu}{KFe}$ | Sélectivité à 10 min $= \frac{[Cu]_{org}}{[Fe]_{org}}$ |
|---|---|---|---|---|---|---|---|---|---|---|
| - $R_1$ | - $R_2$ | - Z - $R_3$ | 3 min | 10 min | 60 min | 3 min | 10 min | 60 min | | |
| 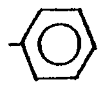 | $-CH_3$ | 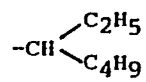 | 69,4% | 75,3% | - | 0,2% | 0,3% | 1,7% | 1013 | 251 |
| 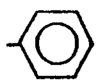 | $-CH_3$ | $-_nC_{11}H_{23}$ | 83% | 86,8% | 86,8% | 0,2% | 0,3% | 0,8% | 2185 | 289 |
| 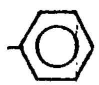 | $-CH_3$ | $-_nC_{17}H_{35}$ * | 55% | 70% | 70% | 0,2% | 0,2% | - | 1160 | 350 |
| 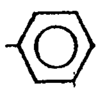 | 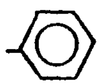 | $-(CH_2)_7-CH=$ $CH-(CH_2)_7-CH_3$ * | 59,4% | 76,8% | 81,5% | 0,05% | 0,1% | 0,2% | 3300 | 768 |
| 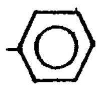 | 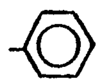 | $-_nC_{11}H_{23}$ | 62,9% | 72,2% | 74,7% | 0,3% | 0,7% | 2% | 370 | 103 |
| 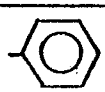 | $-_nC_3H_7$ | $-_nC_{11}H_{23}$ | 48,0% | 66% | - | 0,3% | 0,7% | - | 285 | 94 |
| 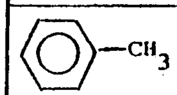 | $-CH_3$ | $-_nC_{11}H_{23}$ * | 42,6% | 65,9% | 88,9% | 0,25% | 1,7% | 1,7% | 111 | 39 |
| LIX 64 N (ESCAID) | | | 78,5% | 83,5% | - | 1,25% | 1,72% | - | 290 | 48 |

* nonyl phénol (10 %)    Solution aqueuse initiale : 3 g/l Cu — 3 g/l Fe.

Tableau V — Extraction du fer seul

temps de contact : 15 minutes

| $R_1$ | $R_2$ | - Z - $R_3$ | Fer extrait | |
|---|---|---|---|---|
| | | | mg phase organique | % d'extraction |
| 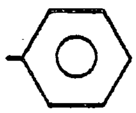 | $-CH_3$ | $-_nC_{11}H_{23}$ | 155 mg | 2% |
| $-_nC_{12}H_{25}$ | $-CH_3$ | $-_nC_{11}H_{23}$ | 648 mg | 8,6% |
| 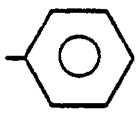 | $-C_3H_7$ | $-_nC_{11}H_{23}$ | 174 mg | 2,3% |
| 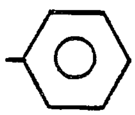 | 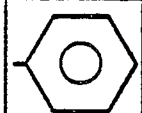 | $-_nC_{11}H_{23}$ | 132 mg | 1,8% |
| 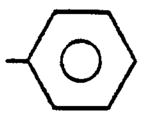 | $-CH_3$ | 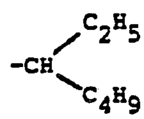 | 121 mg | 1,6% |
| 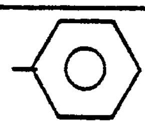 | $-CH_3$ | $-_nC_{17}H_{35}$ | 207 mg | 2,7% |
| 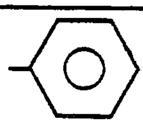 | $-CH_3$ | 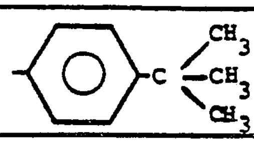 | 648 mg | 8,6% |
| 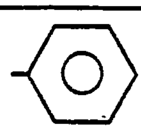 | $-CH_3$ | 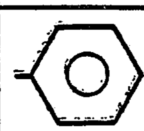 | 2205 mg | 29,4% |

Solution aqueuse initiale : — 7,5 g/l Fe

— pH 2.

## Revendications

1. Procédé pour l'extraction sélective du cuivre de type liquide/liquide, d'une solution aqueuse sulfurique d'attaque du minerai ou d'un semi-produit concentré caractérisé en ce qu'on utilise une solution dans un solvant organique, dissolvant préférentiellement le complexe formé entre l'agent d'extraction et le cuivre, d'au moins un agent d'extraction de formule (A)

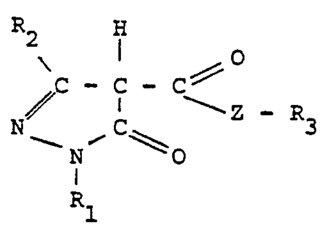 (A)

— où $R_1$ représente un groupe phényle, éventuellement substitué par un ou plusieurs groupes alkyles contenant chacun 1 à 12 atomes de carbone ;

— où $R_2$ représente un groupe alkyle ramifié ou non, contenant 1 à 12 atomes de carbone, un groupe phényle éventuellement substitué par un ou plusieurs groupes alkyles contenant chacun 1 à 12 atomes de carbone ;

— où Z représente un groupe alkylène linéaire ou ramifié, saturé ou insaturé, contenant de 1 à 21 atomes de carbone ;

— où $R_3$ représente H, un (2-phényl 5-alkyl(3H) pyrazolyl-3-one) carbonyl-4 ou un (2-phényl 5-phényl(3H) pyrazolyl-3-one) carbonyl-4 pour former une bis(acyl pyrazolone), les phényles pouvant être substitués ou non par des groupes alkyles, le dit agent pouvant se présenter sous les formes céto-énoliques suivantes correspondant à la formule (A) :

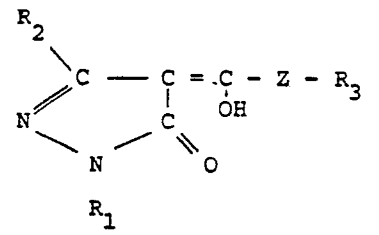

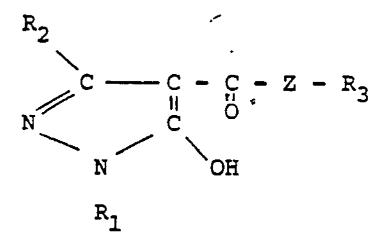

2. Procédé selon la revendication 1, caractérisé en ce qu'on utilise un solvant organique pratiquement insoluble dans l'eau, ne perturbant pas l'action complexante de l'agent d'extraction et ayant un point d'inflammabilité élevé.

3. Procédé selon la revendication 2, caractérisé en ce que le solvant organique est choisi parmi les hydrocarbures aliphatiques ou aromatiques à haut point d'inflammabilité, notamment les kérosènes, les naphtas et analogues.

4. Procédé selon l'une quelconque des revendications précédentes, caractérisé en ce que l'agent d'extraction est à une concentration comprise entre 20 g/l et 750 g/l dans le solvant et de préférence comprise entre 50 g/l et 200 g/l.

5. Procédé selon l'une quelconque des revendications précédentes, caractérisé en ce que la solution d'extraction contient en outre de 2 à environ 20 ml/100 ml de nonylphényl et de préférence environ 10 ml/100 ml.

6. Dérivés de 2-phényl 4-acyl pyrazol-3-one, répondant à la formule (A) de la revendication 1, caractérisés en ce qu'ils sont choisis parmi les composés suivants :

— la di-1,6-(2-phényl-5-méthyl-4-pyrazolyl-3-one)-hexane-1,6-dione

— la 2-phényl-4-(undécénoyl)-5-méthyl-(3H)-pyrazol-3-one

— la 2-phényl-4-(oléoyl)-5-phényl-(3H)-pyrazol-3-one

— la di-1,10-(2-phényl-5-phényl-4-pyrazolyl-3-one)-décane-1,10-dione.

## Claims

1. Liquid/liquid type process for the selective extraction of copper from an aqueous solution formed in the sulphuric acid attack of the ore or of a concentrated partially processed product, characterized in that there is employed a solution in an organic solvent, which preferably dissolves the complex formed between the extraction agent and the copper, of at least one extraction agent of the formula (A)

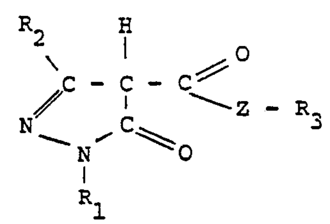

(A)

— where $R_1$ denotes a phenyl group, optionally substituted with one or more alkyl groups each containing 1 to 12 carbon atoms ;

— where $R_2$ denotes a branched or unbranched alkyl group containing 1 to 12 carbon atoms, a phenyl group optionally substituted with one or more alkyl groups each containing 1 to 12 carbon atoms ;

— where Z denotes a saturated or unsaturated, linear or branched alkylene group containing from 1 to 21 carbon atoms ;

— where $R_3$ denotes H, a (2-phenyl-5-alkyl-3-oxo-3H-pyrazol-4-yl) carbonyl or a (2-phenyl-5-phenyl-3-oxo-3H-pyrazol-4-yl) carbonyl so as to form a bis(acylpyrazolone) wherein the phenyl groups may or may

not be substituted by alkyl groups, it being possible for the said agent to take the following ketoenol forms corresponding to the formula (A) :

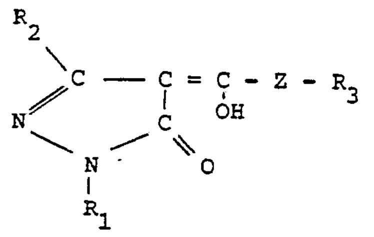
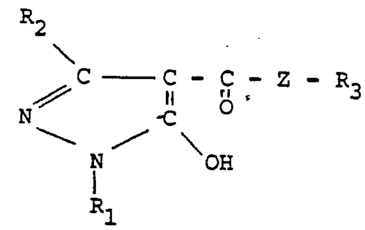

2. Process according to Claim 1, characterized in that an organic solvent is used which is virtually insoluble in water, does not interfere with the complexing action of the extraction agent and has a high inflammation point.

3. Process according to Claim 2, characterized in that the organic solvent is chosen from aliphatic or aromatic hydrocarbons having a high inflammation point, in particular kerosenes, naphthas and the like.

4. Process according to any one of the preceding claims, characterized in that the extraction agent is at a concentration of between 20 g/l and 750 g/l in the solvent, and preferably between 50 g/l and 200 g/l.

5. Process according to any one of the preceding claims, characterized in that the extraction solution contains, in addition, from 2 to approximately 20 ml/100 ml of nonylphenol, and preferably approximately 10 ml/100 ml.

6. 2-Phenyl-4-acyl-3-pyrazolone derivatives corresponding to the formula (A) of Claim 1, characterized in that they are chosen from the following compounds :

— 1,6-bis(2-phenyl-5-methyl-3-oxo-4-pyrazolyl)-1,6-hexanedione
— 2-phenyl-4-undecenoyl-5-methyl-3H-pyrazol-3-one
— 2-phenyl-4-oleoyl-5-phenyl-3H-pyrazol-3-one
— 1,10-bis(2-phenyl-5-phenyl-3-oxo-4-pyrazolyl)-1,10-decanedione.

**Patentansprüche**

1. Verfahren zur selektiven Flüssig-Flüssig-Extraktion von Kupfer aus einer wäßrigen schwefelsauren Aufschlußlösung des Erzes oder aus einem konzentrierten Halbprodukt, gekennzeichnet durch Verwendung einer Lösung in einem organischen Lösungsmittel, das bevorzugt den zwischen dem Extraktionsmittel und dem Kupfer gebildeten Komplex löst, mindestens eines Extraktionsmittels der Formel (A)

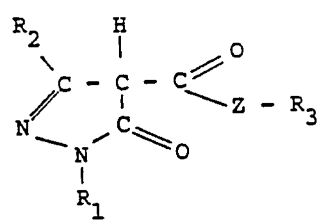

(A)

in der bedeuten

— $R_1$ Phenyl, das ggf. mit mindestens einem $C_{1-12}$-Alkyl substituiert ist,
— $R_2$ geradkettiges oder verzweigtes $C_{1-12}$-Alkyl oder Phenyl, das ggf. mit mindestens einem $C_{1-12}$ Alkyl substituiert ist,
— Z geradkettiges oder verzweigtes, gesättigtes oder ungesättigtes $C_{1-21}$-Alkylen und
— $R_3$ Wasserstoff, (2-Phenyl-5-alkyl-(3H)-pyrazolyl-3-on)-carbonyl-4- oder (2-Phenyl-5-phenyl-(3H)-pyrazolyl-3-on)-carbonyl-4- zur Bildung eines Bis(acylpyrazolons), wobei die Phenylreste ggf. mit Alkylgruppen substituiert sind und das Extraktionsmittel in Form eines Ketoenols entsprechend Formel (A) vorliegen kann :

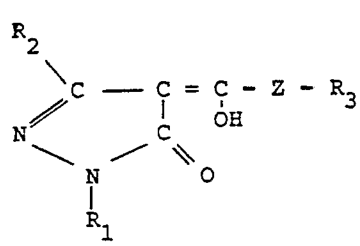
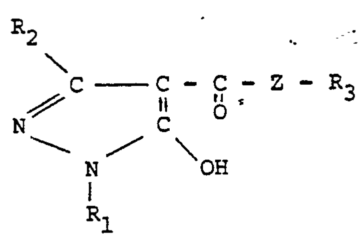

2. Verfahren nach Anspruch 1, gekennzeichnet durch Verwendung eines organischen Lösungsmittels, das in Wasser praktisch unlöslich ist, die komplexbildende Wirkung des Extraktionsmittels nicht stört und einen hohen Flammpunkt hat.

3. Verfahren nach Anspruch 2, gekennzeichnet durch Verwendung eines organischen Lösungsmittels, ausgewählt unter aliphatischen oder aromatischen Kohlenwasserstoffen mit hohem Flammpunkt und insbesondere Kerosinen, Naphtha und Analoga.

4. Verfahren nach einem der vorstehenden Ansprüche, gekennzeichnet durch Verwendung des Extraktionsmittels in einer Konzentration von 20 bis 750 g/l im Lösungsmittel und vorzugsweise von 50 bis 200 g/l.

5. Verfahren nach einem der vorstehenden Ansprüche, gekennzeichnet durch Verwendung einer Extraktionslösung, die außerdem noch 2 bis etwa 20 ml/100 ml und vorzugsweise etwa 10 ml/100 ml Nonylphenyl enthält.

6. 2-Phenyl-4-acylpyrazol-3-on-Derivate der Formel (A) nach Anspruch 1, dadurch gekennzeichnet, daß sie unter folgenden Verbindungen ausgewählt werden :

— Di-1,6-(2-phenyl-5-methyl-4-pyrazolyl-3-on)-hexan-1,6-dion
— 2-Phenyl-4-(undecenoyl)-5-methyl-(3H)-pyrazol-3-on
— 2-Phenyl-4-(oleoyl)-5-phenyl-(3H)-pyrazol-3-on und
— Di-1,10-(2-phenyl-5-phenyl-4-pyrazolyl-3-on)-decan-1,10-dion.

Fig: 1

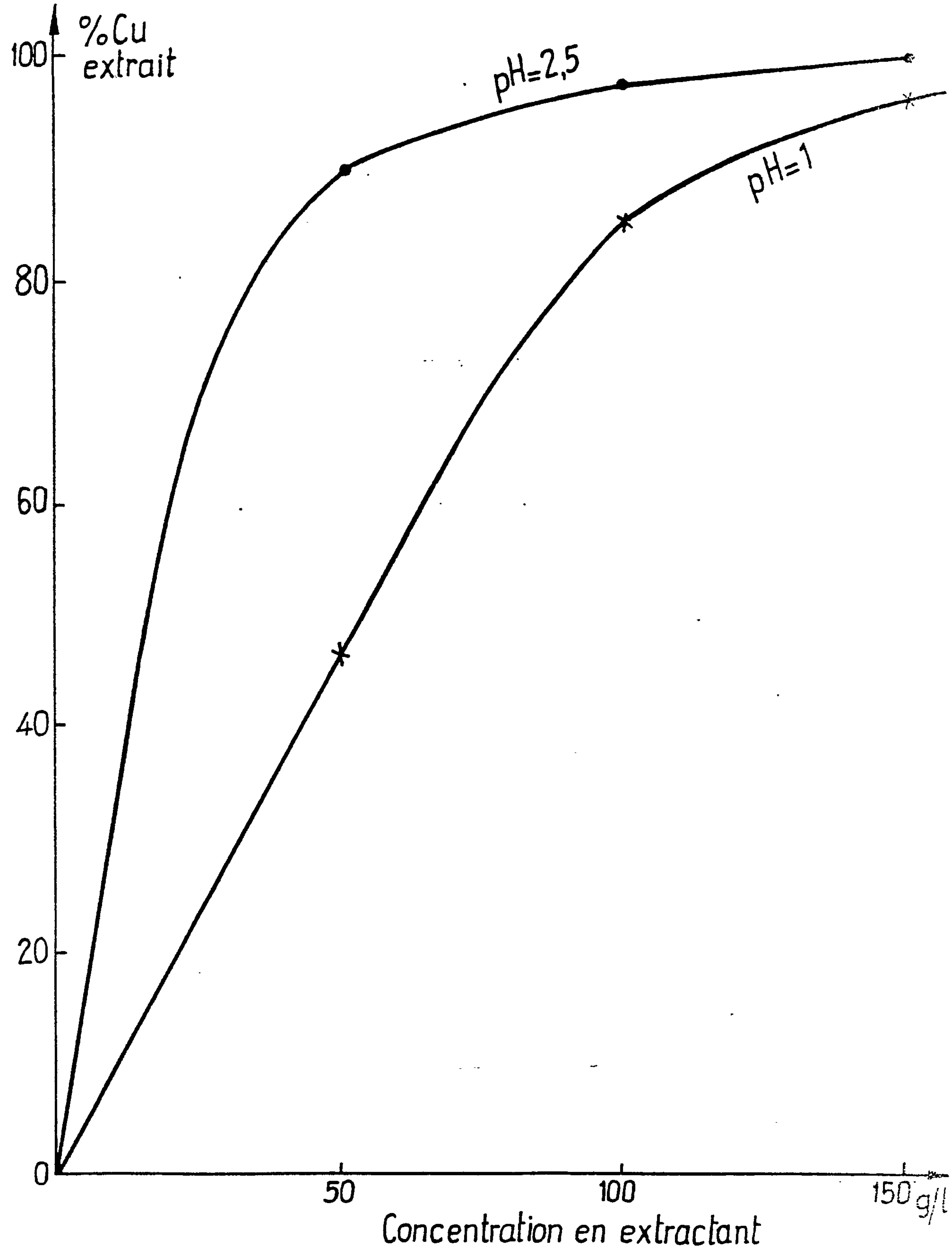
%Cu
extrait
100
80
60
40
20
0
pH=2,5
pH=1
50
100
150 g/l
Concentration en extractant

Fig. 2

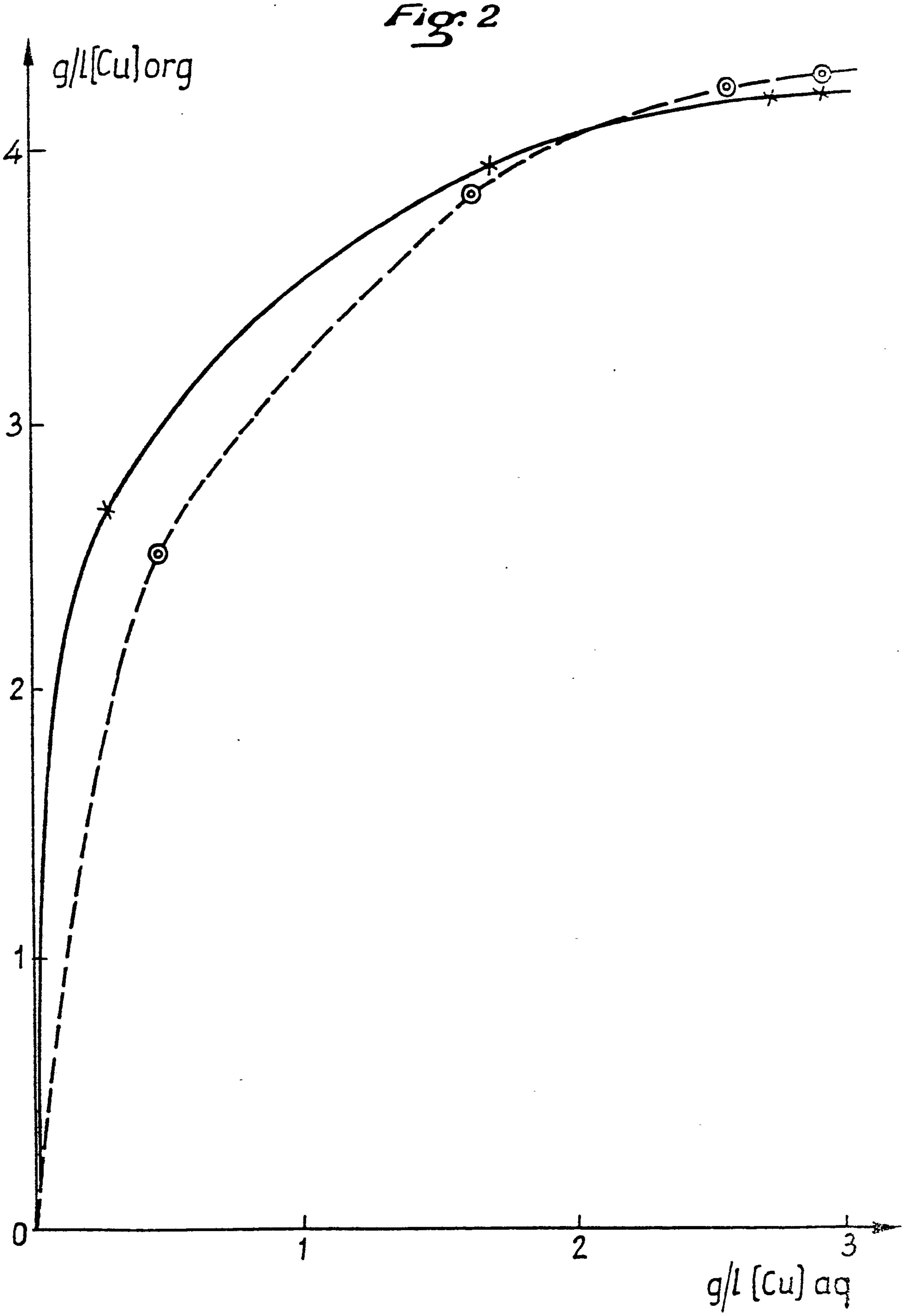

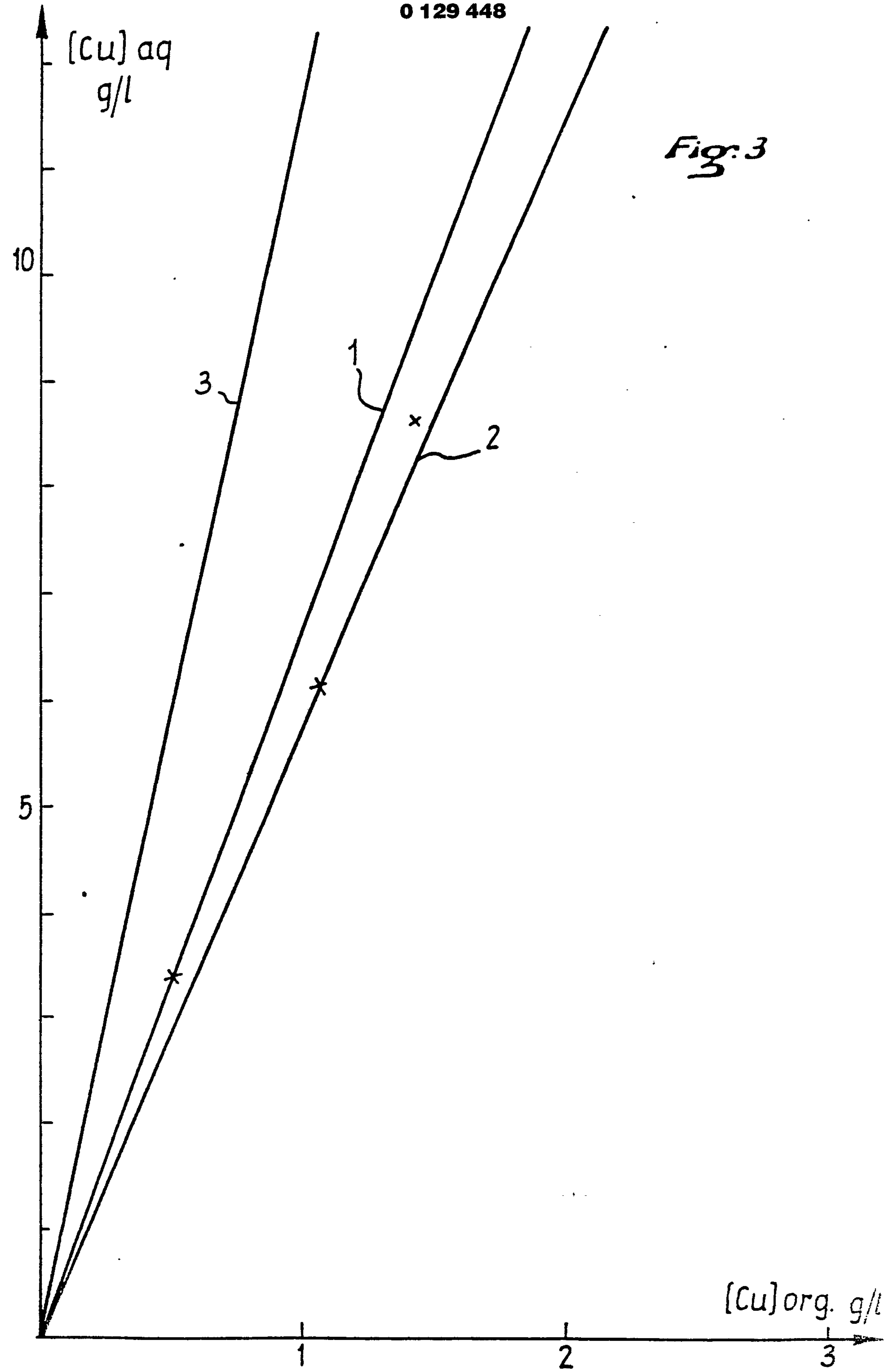


Fig: 3

Fig. 4